# EUROPEAN PATENT SPECIFICATION

(11) **EP 1 737 358 B1**
(45) Date of publication and mention of the grant of the patent: **14.05.2014**
(21) Application number: 05732730.6
(22) Date of filing: 31.03.2005
(51) Int. Cl.: A61B 17/22

(54) **TRACTION CUTTING BALLOON**
SCHNEIDENDER BALLONKATHETER MIT ERHÖHTER TRAKTION
BALLONNET DE COUPE AVEC TRACTION

(30) Priority: 21.04.2004 US 828699
(43) Date of publication of application: 03.01.2007
(73) Proprietor: Boston Scientific Limited, St. Michael (BB)
(72) Inventor: CHEVES, Karen, M., Escondido, CA 92029 (US); CROW, Loren, M., Las Mesa, CA 91941 (US); KELLEY, Gregory, S., San Diego, CA 92119 (US); MCAULEY, Steven, A., Chanhassen, MN 55317 (US); RADISCH, Herbert R., Jr., San Diego, CA 92128 (US); ROMAN, Ricardo, David, San Diego, CA 92139 (US); WU, Show-Mean, San Diego, CA 92129 (US)
(74) Representative: Vossius & Partner
(86) International application number: PCT/US2005/010897
(87) International publication number: WO 2005/107615

(56) References cited:
- WO-A1-2005/020855
- WO-A2-2005/094477
- DE-A1- 3 402 573
- US-A- 5 797 935
- US-A1- 2002 010 489
- US-A1- 2002 151 918
- US-A1- 2003 032 973
- US-B1- 6 306 151

## Description

### Field of the Invention

The present invention pertains to angioplasty and angioplasty balloon catheters. More particularly, the present invention pertains to angioplasty balloon catheters that include one or more cutting edges coupled to the angioplasty balloon.

### Background of the Invention

Heart and vascular disease are major problems in the United States and throughout the world. Conditions such as atherosclerosis result in blood vessels becoming blocked or narrowed. This blockage can result in lack of oxygenation of the heart, which has significant consequences since the heart muscle must be well oxygenated in order to maintain its blood pumping action.

Occluded, stenotic, or narrowed blood vessels may be treated with a number of relatively non-invasive medical procedures including percutaneous transluminal angioplasty (PTA), percutaneous transluminal coronary angioplasty (PTCA), and atherectomy. Angioplasty techniques typically involve the use of a balloon catheter. The balloon catheter is advanced over a guidewire so that the balloon is positioned adjacent a stenotic lesion. The balloon is then inflated and the restriction of the vessel is opened.

One of the major obstacles in treating coronary artery disease and/or treating blocked blood vessels is re-stenosis. Evidence has shown that cutting the stenosis, for example, with an angioplasty balloon equipped with a cutting blade, during treatment can reduce incidence of re-stenosis. Additionally, cutting the stenosis may reduce trauma at the treatment site and/or may reduce the trauma to adjacent healthy tissue. Cutting blades may also be beneficial additions to angioplasty procedures when the targeted occlusion is hardened or calcified. It is believed typical angioplasty balloons, alone, may not be able to expand certain of these hardened lesions. Thus, angioplasty balloons equipped with cutting edges have been developed to attempt to enhance angioplasty treatments. Devices which belong to the state of the art under A7. 54(3) EPC are described in both, WO-A-2005/094477 and WO-A-2005/020855. There is an ongoing need for improved angioplasty devices, including cutting angioplasty balloons, and improved methods of treating intravascular stenoses and occlusions.

### Summary

The present invention relates to angioplasty balloon catheters. In at least some embodiments, an example balloon catheter includes a catheter shaft having a balloon coupled thereto. A cutting member or blade is coupled to the balloon. The cutting member may include one or more traction members or a traction region that can, for example, improve traction between the balloon (or the cutting member) and a target site. These and other features are described in more detail below.

### Brief Description of the Drawings

Figure 1 is partial longitudinal cross-sectional side view of an example catheter disposed in a blood vessel;
Figure 2 is a radial cross-sectional view of an example catheter in the balloon portion where the balloon is partially deflated;
Figure 3 is a partial perspective view of an example cutting member;
Figure 4 is a partial perspective view of another example cutting member;
Figure 5 is a partial perspective view of another example cutting member;
Figure 6 is a partial perspective view of another example cutting member;
Figure 7 is a partial perspective view of another example cutting member; and
Figure 8 is a partial cross-sectional view of another example catheter.

### Detailed Description

The following description should be read with reference to the drawings wherein like reference numerals indicate like elements throughout the several views. The detailed description and drawings illustrate example embodiments of the claimed invention.

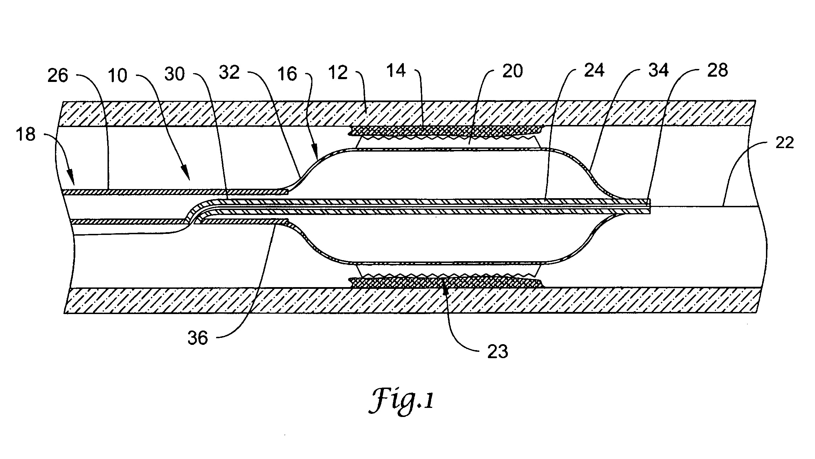

Figure 1 is a partial cross-sectional side view of an example catheter 10 disposed in a blood vessel 12 and positioned adjacent an intravascular lesion 14. Catheter 10 may include a balloon 16 coupled to a catheter shaft 18. In a preferred embodiment, one or more cutting members or blades 20 are coupled to balloon 16. In general, catheter 10 may be advanced over a guidewire 22 through the vasculature to a target area. Balloon 16 can then be inflated to expand lesion 14, and cutting members 20 may cut lesion 14. The target area may be within any suitable peripheral or cardiac location.

Cutting members 20 may help to concentrate force exerted by catheter 10 onto lesion 14 and may cut into or otherwise sever or break up lesion 14. For a number of reasons, it may be desirable for cutting members 20 to also help to increase the traction between catheter 10 and lesion 14. Increasing the traction may help to reduce the possibility that balloon 16 might slip away from lesion 14 during an intervention, which could impact the effectiveness of the intervention. The number, position, and arrangement of cutting members 20 may vary. For example, catheter 10 may include one, two, three, four, five, six, or more cutting members 20 that are disposed at any position along balloon 16 and in a regular, irregular, or any other suitable pattern.

In at least some embodiments, cutting members 20 include a traction member or traction region 23 that may be, for example, adapted and configured to increase traction between catheter 10 (i.e., cutting members 20) and lesion 14. Traction region 23 may vary in its form or structural configuration. For example, traction region 23 may be defined by one or more saw-tooth projections as depicted in Figure 1. This embodiment as well as other embodiments of suitable traction regions 23 is described in more detail below.

Balloon 16 may be made from typical angioplasty balloon materials including polymers such as polyethylene terephthalate (PET), polyetherimid (PEI), polyethylene (PE), etc. Some other examples of suitable polymers, including lubricious polymers, may include polytetrafluoroethylene (PTFE), ethylene tetrafluoroethylene (ETFE), fluorinated ethylene propylene (FEP), polyoxymethylene (POM), polybutylene terephthalate (PBT), polyether block ester, polyurethane, polypropylene (PP), polyvinylchloride (PVC), polyether-ester (for example, a polyether-ester elastomer such as ARNITEL® available from DSM Engineering Plastics), polyester (for example, a polyester elastomer such as HYTREL® available from DuPont), polyamide (for example, DURETHAN® available from Bayer or CRISTAMID® available from Elf Atochem), elastomeric polyamides, block polyamide/ethers, polyether block amide (PEBA, for example, available under the trade name PEBAX®), silicones, Marlex high-density polyethylene, Marlex low-density polyethylene, linear low density polyethylene (for example, REXELL®), polyetheretherketone (PEEK), polyimide (PI), polyphenylene sulfide (PPS), polyphenylene oxide (PPO), polysulfone, nylon, perfluoro(propyl vinyl ether) (PFA), other suitable materials, or mixtures, combinations, copolymers thereof, polymer/metal composites, and the like. In some embodiments, it may be desirable to use high modulus or generally stiffer materials so as to reduce balloon elongation. The above list of materials includes some examples of higher modulus materials. Some other examples of stiffer materials include polymers blended with liquid crystal polymer (LCP) as well as the materials listed above. For example, the mixture can contain up to about 5% LCP.

Shaft 18 may be a catheter shaft, similar to typical catheter shafts. For example, shaft 18 may include an inner tubular member 24 and outer tubular member 26. Tubular members 24/26 may be manufactured from a number of different materials. For example, tubular members 24/26 may be made of metals, metal alloys, polymers, metal-polymer composites or any other suitable materials. Some examples of suitable metals and metal alloys include stainless steel, such as 300 series stainless steel (including 304V, 304L, and 316L; 400 series martensitic stainless steel; tool steel; nickel-titanium alloy such as linear-elastic or super-elastic Nitinol, nickel-chromium alloy, nickel-chromium-iron alloy, cobalt alloy, tungsten or tungsten alloys, MP35-N (having a composition of about 35% Ni, 35% Co, 20% Cr, 9.75% Mo, a maximum 1% Fe, a maximum 1% Ti, a maximum 0.25% C, a maximum 0.15% Mn, and a maximum 0.15% Si), hastelloy, monel 400, inconel 825, or the like; or other suitable material. Some examples of suitable polymers include those described above in relation to balloon 16. Of course, any other polymer or other suitable materials including ceramics may be used without departing from the the invention. The materials used to manufacture inner tubular member 24 may be the same as or be different from the materials used to manufacture outer tubular member 26. Those materials listed herein may also be used for manufacturing other components of catheter 10 including cutting members 20.

Tubular members 24/26 may be arranged in any appropriate way. For example, in some embodiments inner tubular member 24 can be disposed coaxially within outer tubular member 26. According to these embodiments, inner and outer tubular members 24/26 may or may not be secured to one another along the general longitudinal axis of shaft 18. Alternatively, inner tubular member 24 may follow the inner wall or otherwise be disposed adjacent the inner wall of outer tubular member 26. Again, inner and outer tubular members 24/26 may or may not be secured to one another. For example, inner and outer tubular members 24/26 may be bonded, welded (including tack welding or any other welding technique), or otherwise secured at a bond point. In some embodiments, the bond point may be generally disposed near the distal end of shaft 18. However, one or more bond points may be disposed at any position along shaft 18. The bond may desirably impact, for example, the stability and the ability of tubular members 24/26 to maintain their position relative to one another. In still other embodiments, inner and outer tubular member 24/26 may be adjacent to and substantially parallel to one another so that they are non-overlapping. In these embodiments, shaft 18 may include an outer sheath that is disposed over tubular members 24/26.

Inner tubular member 24 may include an inner lumen 28. In a preferred embodiment, inner lumen 28 is a guidewire lumen. Accordingly, catheter 10 can be advanced over guidewire 22 to the desired location. The guidewire lumen may extend along essentially the entire length of catheter shaft 18 so that catheter 10 resembles traditional "over-the-wire" catheters. Alternatively, the guidewire lumen may extend along only a portion of shaft 18 so that catheter 10 resembles "single-operator-exchange" or "rapid-exchange" catheters. Regardless of which type of catheter is contemplated, catheter 10 may be configured so that balloon 16 is disposed over at least a region of inner lumen 28. In at least some of these embodiments, inner lumen 28 (i.e., the portion of inner lumen 28 that balloon 16 is disposed over) may be substantially coaxial with balloon 16.

Shaft 18 may also include an inflation lumen 30 that may be used, for example, to transport inflation media to and from balloon 16. The location and position of inflation lumen 30 may vary, depending on the configuration of tubular members 24/26. For example, when outer tubular member 26 is disposed over inner tubular member 24, inflation lumen 30 may be defined within the generally annular space between tubular members 24/26. Moreover, depending on the position of inner tubular member 24 within outer tubular member 26, the shape of lumen 30 (i.e., the shape adjacent shaft 18) may vary. For example, if inner tubular member 24 is attached to or disposed adjacent to the inside surface of outer tubular member 26, then inflation lumen 30 may be generally half-moon in shape; whereas if inner tubular member 24 is generally coaxial with outer tubular member 26, then inflation lumen 30 may be generally ring-shaped or annular in shape. It can be appreciated that if outer tubular member 26 is disposed alongside inner tubular member 24, then lumen 30 may be the lumen of outer tubular member 26 or it may be the space defined between the outer surface of tubular members 24/26 and the outer sheath disposed thereover.

Balloon 16 may be coupled to catheter shaft 18 in any of a number of suitable ways. For example, balloon 16 may be adhesively or thermally bonded to shaft 18. In some embodiments, a proximal waist 32 of balloon 16 may be bonded to shaft 18, for example, at outer tubular member 26, and a distal waist 34 may be bonded to shaft 18, for example, at inner tubular member 24. The exact bonding positions, however, may vary. It can be appreciated that a section of proximal waist 32 may not have sections 36 extending therefrom in order for suitable bonding between balloon 16 and outer tubular member 30.

In addition to some of the structures described above, shaft 18 may also include a number of other structural elements, including those typically associated with catheter shafts. For example, shaft 18 may include a radiopaque marker coupled thereto that may aid a user in determining the location of catheter 10 within the vasculature. In addition, catheter 10 may include a folding spring (not shown) coupled to balloon 16, for example, adjacent proximal waist 32, which may further help in balloon folding and refolding. A description of a suitable folding spring can be found in U.S. Patent No. 6,425,882.

As stated above, cutting members 20 may include traction region 23, which may have a number of different forms or configurations. The embodiment depicted in Figure 1 illustrates that traction region 23 may be defined by a number of saw-tooth projections on a cutting blade that is affixed longitudinally on the balloon 16. The shape, pattern, configuration, and number of the projections can vary. For example, the projections shown in Figure 1 have a generally pointed shape. But, any suitable shape may be used without departing from the invention. For example, the projections may be squared, polygonal, rounded, etc. In addition, although the traction region 23 is depicted as being aligned with the longitudinal axis of cutting members 20 and extending outward therefrom, this arrangement is not intended to be limiting. It can be appreciated that traction region 23 may be disposed along any portion of cutting members 20 and in any suitable arrangement. For example, some of the saw tooth projections may extend laterally from the longitudinal axis of cutting members 20.

The saw-toothed configuration of traction region 23 allows cutting members 20 to more tightly grip lesion 14 and/or to more deeply penetrate into lesion 14. Accordingly, cutting members 20 may be more tightly anchored when they are engaged with lesion 14. This can improve the traction and/or positional stability of catheter 10 during an intervention. In addition, the saw-toothed configuration reduces the surface area of cutting members 20 at the point of contact. This allows cutting members 20 to effectively engage lesion 14.

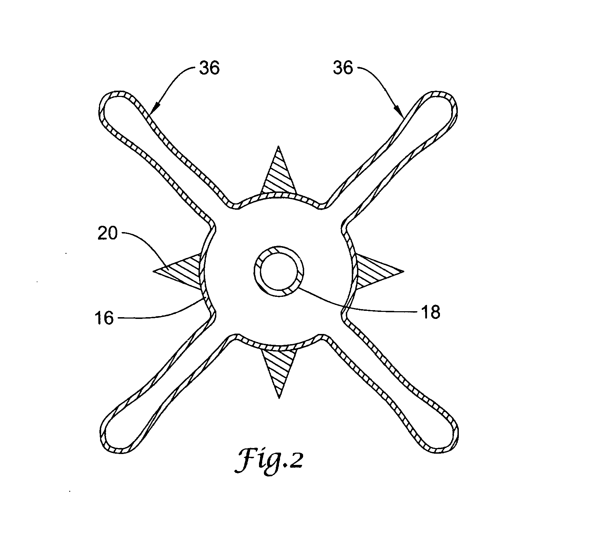

Balloon 16 may be configured so that it includes one or more wings 36, as shown in Figure 2. In general, wings 36 are visible and can be seen when balloon 16 is deflated. The appearance of wings 36 includes a plurality of alternating inward and outward radial deflection in balloon 16. Wings 36 may allow balloon 16 to have more predictable and consistent re-folding characteristics. For example, wings 36 may help balloon 16 fold inward at a plurality of positions so that the overall profile of balloon 16 in a deflated state can be reduced. In some embodiments, balloon 16 includes four wings 36. However, the number of wings 36 can vary and can be any suitable number such as three, four five, six, or more. The distribution of wings 36 may also vary. For example, wings 36 may be evenly, regularly, irregularly, randomly, or otherwise dispersed in any manner about balloon 16.

In at least some embodiments, wings 36 may be dispersed so that wings 36 and cutting members 20 alternate. Additionally, it may be desirable to configure wings 36 so that cutting members 20 are positioned at the inward-most positions of wings 36. This arrangement allows cutting members 20 to be positioned more closely to shaft 18 when balloon 16 is deflated. Accordingly, cutting members 20 can be moved away from the vessel walls where they might otherwise result in contact and, possibly, damage to healthy tissue during movement of catheter 10 within a body lumen. Additionally, alternating wings 36 and cutting members 12 as well as positioning cutting members 20 relatively close to shaft 18 may allow wings 36 to fold over and cover cutting members 20 when balloon 16 is deflated. Again, this feature may reduce the exposure of cutting members 20 to the blood vessel.

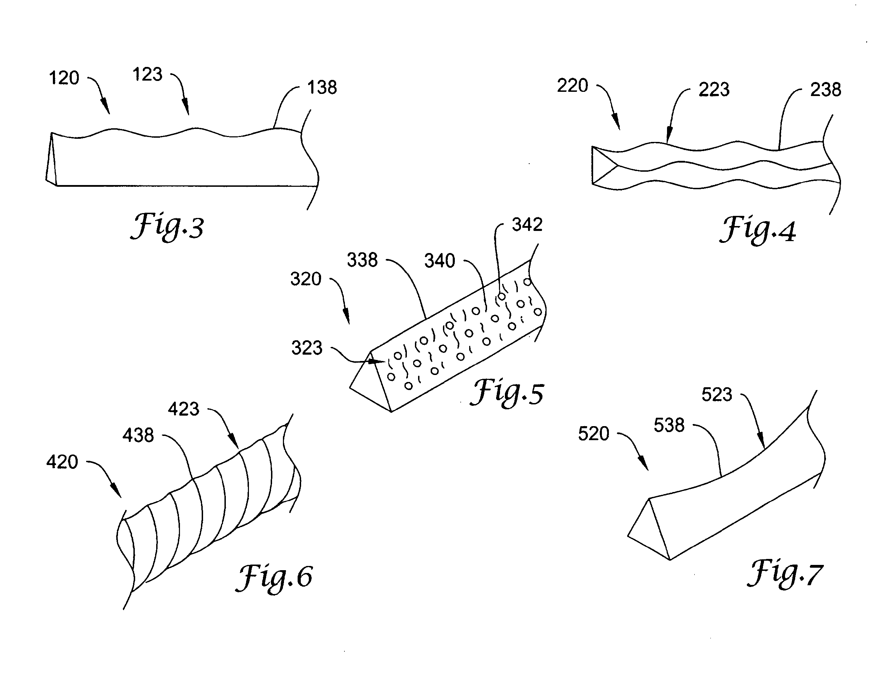

Another example cutting member 120 is illustrated in Figure 3 that can be used with catheter 10 or any other suitable device. Cutting member 120 is similar in form and function to cutting member 20 except that traction region 123 is defined by a plurality of undulations in the top or cutting surface 138 of cutting member 120. The undulations define a "wavy" or "curvy" top surface 138 with a varying height. Accordingly, cutting member 120 can be thought of as being similar in shape to cutting members 20 except that the transition between adjacent "peaks" or "teeth" is more gradual than in cutting members 20. A number of variations in the shape and configuration of traction region 123 are contemplated. For example, the curves in traction region 123 may have a varying slope or radius of curvature, be spaced out regularly or irregular, be constant or intermittent, or have any other suitable arrangement.

Cutting member 120 functions similarly to cutting members 20. For example, cutting member 120 can be coupled to balloon 16 in essentially the same manner as cutting members 20 and can be dispersed or arranged in any suitable manner. Upon inflation of balloon 16, cutting members 120 can cut into and/or sever lesion 14. Traction region 123 can grip lesion 14 (in a manner similar to how cutting members 20 can grip lesion 14 as described above) so that the position of balloon 16 and/or cutting member or members 120 can remain essentially stable.

Another example cutting member 220 is illustrated in Figure 4, where the curves or undulations are "side-to-side" (rather than "up-and-down") so as to define traction region 223. In Figure 4, cutting member 220 has been rotated slightly in order to more clearly show traction region 223. In a manner similar to how cutting member 120 functions, cutting member 220 also can improve traction. For example, traction region 223 may improve traction by increasing the contacting surface area between cutting member 220 and lesion 14.

Figure 5 illustrates an enlarged view of cutting member 320 that includes a traction region 323 that is defined by a textured surface 340 and/or series of bumps or projections 342 disposed along cutting member 320. Textured surface 340 can be formed or defined in any suitable manner. For example, textured surface 340 can be formed by scoring, grinding, scuffing, or otherwise altering cutting member 320. The pattern of textured surface 340 may also vary and can be random, regular, intermittent, or any other suitable pattern.

Similarly, bumps 342 may be formed, defined, or attached to cutting member 320 in any suitable manner. For example, bumps 342 (and/or textured surface 340) may be defined by grinding cutting member 320. Alternatively, bumps 342 may be molded, bonded, or otherwise attached to traction member in any suitable way. The pattern may also be random, regular, or intermittent. Bumps 342 may have any suitable shape. For example, bumps may be rounded or cylindrical, squared, triangular or pyramidal, polygonal, pointed, blunted, and the like, or any other suitable shape.

In general, traction region 323 (i.e., textured surface 340 and/or bumps 342 that define traction region 323) may be disposed along the entire length of cutting member 320 or along any portion thereof. Traction region 323 need not be disposed in a continuous arrangement and may be disposed intermittently or in any other suitable arrangement. For example, traction region 323 may include textured surface 340 without bumps 342 followed by textured surface 340 with bumps 342, with or without a space therebetween. The position of textured surface 340 and/or bumps 342 may also be relative to top or cutting surface 338. For example, in some embodiments textured surface and/or bumps 342 may be disposed relatively close to cutting surface 338, while in other embodiments some degree of spacing may occur between these structures.

Figure 6 illustrates cutting member 420 having traction region 423 that is defined by a twist or helical winding formed in cutting member 420. The twist or winding may define a series of peaks or ridges that define cutting surface 438. The twist defining traction region 423 can be formed in traction member 120 in any suitable manner and may be continuous, intermittent, have a regular or irregular pitch, or configured in any suitable manner. Traction region 423 of helically oriented cutting member 420 may help improve traction in a manner similar to what is described above. For example, traction region 423 may increase the surface area of contact between cutting member 420 and lesion 14.

Cutting member 520 is shown in Figure 7 and may include a saddle-shaped traction region 523. According to this embodiment, cutting surface 538 may bend or curve inward. In a manner similar to what is described above, traction region 523 may help improve traction between cutting member 520 and lesion 14.

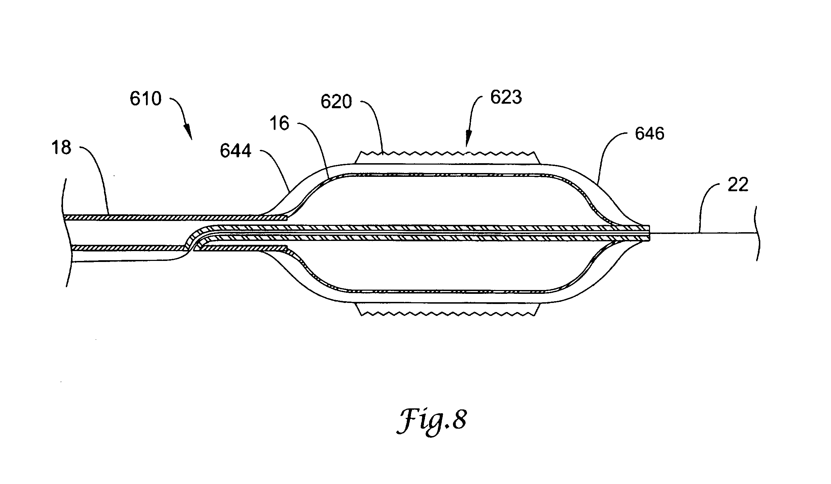

In at least some embodiments, any of the cutting members described herein may be coupled to balloon 14 by adhesive bonding, thermal bonding, welding, and the like as described above. However, other embodiments are contemplated that utilize alternative arrangements. For example, Figure 8 illustrates catheter 610 that includes a plurality of "floating" cutting members 620. These cutting members 620 may be similar to any of the other cutting members disclosed herein (i.e., they may include traction region 623) and may be disposed alongside balloon 16 and attached to balloon 16 and/or shaft 18 with flanking proximal and distal connectors 644/646, respectively. Connectors 644/646 could be a shaft or wire attached to cutting members 620 and/or shaft 18. Alternatively, connectors 644/646 could include a mesh, matrix, or any other suitable structure.

Cutting members 620 may be configured to improve traction, thus holding the position of catheter 610, while still allowing the shape or position of balloon 16 to vary somewhat. This feature may be desirable for a number of reasons. For example, anchoring cutting members 620 independently of balloon 16 may allow balloon 16 to shift in order to expand lesions that may shift or move during the intervention. In addition, manufacturing of catheter 610 may be simplified by allowing for the attachment of cutting members by simply attaching connectors 644/646. In some embodiments, connectors 644/646 may be mechanically connected to shaft 18, balloon 16, or both by winding connectors 644/646 about the relevant structure. However, any other suitable attachment method may be used without departing from the invention.

It should be understood that this disclosure is, in many respects, only illustrative. Changes may be made in details, particularly in matters of shape, size, and arrangement of steps without exceeding the scope of the invention. The invention's scope is, of course, defined in the language in which the appended claims are expressed.

## Claims

1. A medical device (10), comprising:
an elongate shaft (18) having a proximal end, a distal end, an inner lumen (28) extending therethrough, and an inflation lumen (30) extending therethrough;
a balloon (16) coupled to the shaft (18); and
one or more cutting members (120, 220) coupled to the balloon (16),
wherein
the one or more cutting members (120, 220) each include a traction region (123, 223) that improves traction between the balloon (16) and a target site, the traction region being defined by a series of undulations in the cutting members curving from side-to-side to increase traction between the cutting members and a lesion when the balloon is inflated to expand the lesion; and
the balloon (16) is configured to include one or more wings (36) alternating with the cutting members (120, 220) about the balloon (16) when the balloon (16) is deflated, the wings including a plurality of alternating inward and outward radial deflections in the balloon when the balloon is deflated.

2. The medical device of claim 1, wherein the undulations curve up and down.

3. The medical device of claim 1, wherein the cutting members (120, 220) each include a proximally-extending connector (644) and a distally-extending connector (646) that are both attached to the shaft (18).

4. The medical device of claim 3, wherein the proximally-extending connector (644) and the distally-extending connector (644) are connected to the shaft (18) at opposing sides of the balloon (16).

5. The medical device of claim 4, wherein the cutting members (120, 220) are not directly attached to the balloon (16).

## Patentansprüche

1. Medizinprodukt (10), das aufweist:
einen länglichen Schaft (18) mit einem proximalen Ende, einem distalen Ende, einem Innenlumen (28), das sich durch ihn erstreckt, und einem Inflationslumen (30), das sich durch ihn ersttreckt;
einen Ballon (16), der mit dem Schaft (18) gekoppelt ist; und
ein oder mehrere Schneidteile (120, 220), die mit dem Ballon (16) gekoppelt sind, wobei
das eine oder die mehreren Schneidteile (120, 220) jeweils einen Traktionsbereich (123, 223) aufweisen, der die Traktion zwischen dem Ballon (16) und einer Zielstelle verbessert, wobei der Traktionsbereich durch eine Folge von Welligkeiten in den Schneidteilen gebildet ist, die sich von einer Seite zur anderen krümmen, um die Traktion zwischen den Schneidteilen und einer Läsion zu erhöhen, wenn der Ballon inflatiert wird, um die Läsion zu expandieren; und
der Ballon (16) so konfiguriert ist, dass er einen oder mehrere Flügel (36) aufweist, die sich mit den Schneidteilen (120, 220) um den Ballon (16) abwechseln, wenn der Ballon (16) deflatiert ist, wobei die Flügel mehrere alternierende radiale Einwärts- und Auswärtsablenkungen im Ballon aufweisen, wenn der Ballon deflatiert ist.

2. Medizinprodukt nach Anspruch 1, wobei sich die Welligkeiten nach oben und unten krümmen.

3. Medizinprodukt nach Anspruch 1, wobei die Schneidteile (120, 220) jeweils einen sich proximal erstreckenden Verbinder (644) und einen sich distal erstreckenden Verbinder (646) aufweisen, die beide am Schaft (18) angebracht sind.

4. Medizinprodukt nach Anspruch 3, wobei der sich proximal erstreckende Verbinder (644) und der sich distal erstreckende Verbinder (646) mit dem Schaft (18) an gegenüberliegenden Seiten des Ballons (16) verbunden sind.

5. Medizinprodukt nach Anspruch 4, wobei die Schneidteile (120, 220) nicht direkt am Ballon (16) angebracht sind.

## Revendications

1. Dispositif médical (24), comprenant :
une tige oblongue (18) avec une extrémité proximale, une extrémité distale, une lumière intérieure (28) s'étendant entre celles-ci, et une lumière de gonflage (30) s'étendant entre celles-ci ;
un ballonnet (16) relié à la tige (18) ; et
un ou plusieurs éléments de coupe (120, 220) reliés au ballonnet (16),
l'élément ou les éléments de coupe (120, 220) présentant chacun une zone de traction (123, 223) améliorant la traction entre le ballonnet (16) et un site cible, la zone de traction étant définie par une série d'ondulations dans les éléments de traction, s'étendant de part et d'autre pour accroître la traction entre les éléments de traction et une lésion quand le ballonnet est gonflé pour distendre la lésion ; et
le ballonnet (16) étant prévu pour comporter une ou plusieurs ailettes (36) alternées avec les éléments de coupe (120, 220) autour du ballonnet (16) quand le ballonnet est dégonflé, lesdites ailettes présentant une pluralité de déflections radiales intérieures et extérieures alternées sur le ballonnet quand le ballonnet est dégonflé.

2. Dispositif médical selon la revendication 1, où les ondulations s'étendent vers le haut et vers le bas.

3. Dispositif médical selon la revendication 1, où les éléments de coupe (120, 220) comportent chacun un connecteur à extension proximale (644) et un connecteur à extension distale (646) tous deux fixés sur la tige (18).

4. Dispositif médical selon la revendication 3, où le connecteur à extension proximale (644) et le connecteur à extension distale (646) sont raccordés à la tige (18) sur des côtés opposés du ballonnet (16).

5. Dispositif médical selon la revendication 4, où les éléments de coupe (120, 220) ne sont pas directement fixés sur le ballonnet (16).
